# EUROPEAN PATENT APPLICATION

(11) **EP 4 052 716 A1**
(43) Date of publication of application: **07.09.2022**
(21) Application number: 21160732.0
(22) Date of filing: 04.03.2021
(51) Int. Cl.: A61K 35/17, A61K 35/768, A61P 35/00, C07K 14/725, C07K 16/30, C12N 5/0783, A61K 39/00

(54) **CANCER THERAPY INVOLVING CAR-ENGINEERED T-CELLS AND PARVOVIRUS H-1**

(71) Applicant: Deutsches Krebsforschungszentrum - Stiftung des öffentlichen Rechts / Universität Heidelberg, 69120 Heidelberg (DE)
(72) Inventor: The designation of the inventor has not yet been filed
(74) Representative: Schüssler, Andrea

(57) **Abstract**

The present invention relates to compositions, methods, uses and kits for combination therapies involving immunotherapies, such as adaptive cell therapy, e.g., T cell therapy, and an oncolytic virus (particularly parvovirus H-1), for treating subjects with cancer. The T cell therapy includes cells that express recombinant receptors such as chimeric antigen receptors (CARs). In some embodiments, the cancer is a solid tumor or a hematological malignancy.

## Description

The present invention relates to compositions, methods, uses and kits for combination therapies involving immunotherapies, such as adaptive cell therapy, e.g., T cell therapy, and an oncolytic virus (particularly parvovirus H-1), for treating subjects with cancer. The T cell therapy includes cells that express recombinant receptors such as chimeric antigen receptors (CARs). In some embodiments, the cancer is a solid tumor or a hematological malignancy.

### Background of the Present Invention

For many years cancer treatment was based on surgery, chemotherapy, and radiation, and more recently targeted therapies. Although these approaches have contributed to improved outcomes, most malignancies still have a poor prognosis. Targeted anticancer approaches provide individualized therapy to combat the complexity of most malignancies and increase the probability of success. Currently, immunotherapies which use the power of a patient's immune system to fight disease have gained increased interest. One approach to cancer immunotherapy entails genetically engineering a patient's T cells to express chimeric antigen receptors (CARs) that recognize and attack tumor cells. The CAR consists of an antibody or ligand-derived targeting ectodomain fused with a hinge, a trans-membrane domain, and intracellular T cell signaling domains. When expressed by a T cell, CARs confer antigen specificity determined by the targeting domain. In contrast to conventional T cell receptors (TCRs), which recognize antigens in a major histocompatibility complex (MHC)-dependent manner, CARs can potentially redirect the effector functions of a T cell toward any protein or non-protein target expressed on the cell surface. This strategy thereby avoids the requirement of antigen processing and presentation by the target cell and is applicable to non-classical T cell targets. Circumventing human MHC-restriction renders the CAR-T cell approach as a universal treatment, broadening the potential applicability of adoptive T cell therapy.

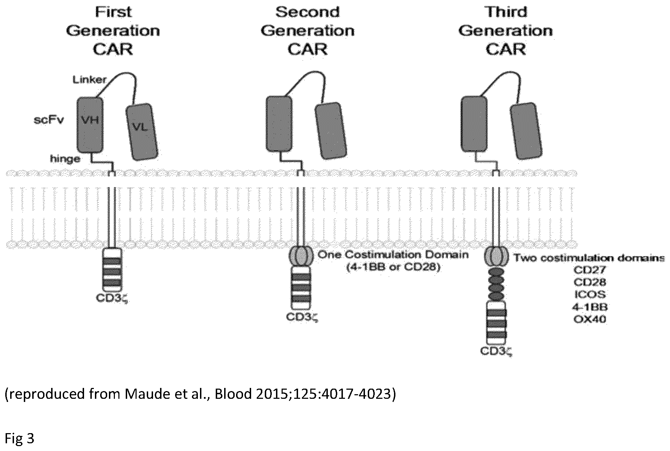

Four generations of CAR are being investigated in preclinical and ongoing clinical studies (Mirzaei et al., Frontiers in Immunology 2017, Vol. 8, Art. 1850). The CAR "generation" typically refers to the intracellular signaling domains incorporated in the receptor molecule. First-generation CARs include only CD3ζ as an intracellular signaling domain; second-generation CARs include in addition to CD3ζ, a single co-stimulatory domain, such as CD28, 4-1BB (CD137), CD27, or OX40; third-generation CARs contain CD3ζ and two co-stimulatory domains, such as CD28, 4-1BB, or other co-stimulatory molecules (c.f. Fig. 3). CARs may be further manipulated through the introduction of additional genes, including those encoding potent antitumor cytokines (e.g., IL-12 and 11-15) or co-stimulatory ligands (e.g., 4-1BBL), thus producing "armored" fourth-generation CAR T-cells (Maus et al., Blood 2014, 123(1), 2625-2635; Pegram et al., Cancer J. 2014, 20(2): 127).

Chimeric antigen receptors targeting the B cell receptor-associated protein CD19, developed for the treatment of B cell leukemia and lymphomas, have been the most clinically tested to date. Much progress with CD19-CAR T cell therapy across multiple institutions employing different therapeutic designs has led to the successful commercialization of this adoptive immunotherapy. Two CD19-targeting CAR-T cell products, Kymriah^{®} (Tisagenlecleucel) from Novartis (East Hanover, NJ, USA) and Yescarta^{®} (Axicabtagene ciloleucel) from Kite Pharma (Santa Monica, CA, USA), have been approved in 2017 by the US FDA for treating B cell acute lymphoblastic leukemia (B-ALL) and diffuse large B-cell lymphoma (DLBCL), respectively. The CAR-T cell therapy has achieved remarkable outcomes in children and young adults with relapsed and often refractory disease, with complete response (CR) rates of 70-90% (Cummins et al., Leuk. Lymphoma 2017, 1-15). In lymphomas and other B cell malignancies, CAR T cell therapy, while effective, has shown lower CR rates, near 55% (Cummins et al., Leuk. Lymphoma 2017, 1-15). Both FDA-approved CARs specifically bind CD19, an antigen that works well as a target for hematological malignancies because it is nearly uniformly expressed on malignant cells and appears on all B cells, both healthy and malignant. Thus, CD19-CAR-T cell treatment may cause B cell aplasia, but the condition can be managed with intravenous immunoglobulins and close infection monitoring.

Despite progress in the treatment of hematological cancers with CAR-T cells, treatment of solid tumors has proven more difficult. The limited success of CAR-T cell therapy against solid tumors may be due to many factors, including: (i) the lack of a unique tumor-associated antigen (TAA) in most cancers; (ii) the inability of *ex vivo* expanded CAR-T cells to persist and proliferate following adoptive transfer; (iii) inefficient trafficking of CAR-T cells to tumor sites; (iv) heterogeneous expression of the targeted antigen(s) leading to outgrowth of antigen-negative tumor variants; (v) the lack of survival and growth factors (e.g., IL-2); (vi) the presence of immunosuppressive molecules and cells; and (vii) the metabolically hostile tumor microenvironment (Zhang et al., Int. J. Biol. Sci. 2016, 12:718-729). Many strategies and approaches have been tried to overcome these obstacles, including arming CAR-T cells with knock-out of PD-1 expression or secretion of cytokines/chemokines and using CAR-T cells in combination with checkpoint inhibitors (Heczey et al., Mol. Ther. 2017, 25: 2214-2224; Rupp et al., Sci. rep 2017, 7:737; Hedge et al., Cancer Immunol. Immunother. 2017, 66:1113-1121). Despite these efforts, there are still no CAR-T cells clinically approved for solid tumor treatment so far although several clinical studies are ongoing.

### Object

Thus, improved strategies are needed to improve efficacy of the CAR-T cells for treating solid tumors. These improved strategies may involve the improving of the persistence, activity and/or proliferation of the cells upon administration to subjects.

### Detailed Description of the Present Invention

According to the invention this is achieved by the subject matters defined in the claims.

The present inventors were successful in showing that a combined use of (a) immune cells (particularly T cells), genetically modified to express a chimeric antigen receptor (CAR) [in the following "CAR cells"] and (b) an oncolytic virus (particularly parvovirus H-1) improves the efficacy of tumor treatment, particularly for treating solid tumors. Thus, the present invention concerns a pharmaceutical combination or medical preparation comprising (a) T cells genetically modified to express a chimeric antigen receptor (CAR) and (b) parvovirus H-1.

The still poor outcome of immunotherapies, including CAR-T technology, for treating solid tumors is caused by a barrier around solid tumors which makes the invasion of T cells difficult or even impossible. This barrier is called the "tumor microenvironment" (TME) and is the cellular environment in which the tumor exists, including surrounding blood vessels, immune cells, fibroblasts, bone marrow- derived inflammatory cells, lymphocytes, signaling molecules and the extracellular matrix. The tumor and the surrounding microenvironment are closely related and interact constantly. Tumors can influence the microenvironment by releasing extracellular signals, promoting tumor angiogenesis and inducing peripheral immune tolerance, while the immune cells in the microenvironment can affect the growth and evolution of cancerous cells. Clinical and preclinical studies have shown that reversing immune inhibitory pathways triggered in many cancers may require CAR T cell modifications by e.g. inclusion of co-stimulatory signaling or additional active agents. In contrast to certain blood cancers that have responded well to CAR-T cell therapy, solid tumors not only lack conventional co-stimulatory molecules, which are expressed on malignant and normal B lymphocyte targets in hematological malignancies, but also have evolved mechanisms to actively suppress the immune system. A number of immunosuppressive pathways can limit the full potential of adoptive CAR T cell therapy. Inhibitory immune receptors are often expressed on T cells following persistent tumor antigen encounter, and these include T-cell membrane protein-3 (TIM-3), lymphocyte-activation protein-3 (LAG-3), T cell Ig and ITIM domain (TIGIT), cytotoxic T lymphocyte-associated antigen 4 (CTLA-4), and programmed death-1 (PD-1). The upregulation of these receptors limit the persistence and activity of the antitumor response of CAR T cells. Thus, tumors employ multiple tactics to evade or misdirect tumor-specific immune response.

Thus, the present inventors combined CAR-T cell technology with the oncolytic virus parvovirus H-1 to convert so-called "cold tumors", i.e. tumors with a low degree of immune cell infiltration, into "hot tumors", which are immunogenic tumors, i.e. with an intermediate or high degree of immune cell infiltration. The concept of "cold" and "hot" tumors is well known to the person skilled in the art. Cold tumors are usually enriched in immunosuppressive cytokines and have high numbers of Treg cells and myeloid-derived suppressor cells (MDSC). Cold tumors usually have few numbers of TH 1 cells, NK cells and CD8+ T cells and few functional antigen-presenting cells (APC) (for example dendritic cells (DC)). In contrast, hot tumors are enriched in TH 1-type chemokines and have high numbers of effector immune cells (TH 1 cells, NK cells and CD8+ T cells) and high numbers of DC. The chemokines CXCL9, CXCL10 and CX3CL1 play an important role in the attraction of T cells in many cancer types.

The degree of immune cell infiltration can, for example, be measured by the so-called "immunoscore", which is used to predict clinical outcome in patients with cancer. The consensus immunoscore is a scoring system to summarize the density of CD3+ and CD8+ T-cells within the tumor and its invasive margin. For example, the immunoscore can be classified as low, intermediate and high depending on the CD3+ / CD8+ T cell density, whereas a 0-25 % density is preferably scored as low, a 25-70 % density is preferably scored as intermediate and a 70-100 % density is preferably scored as high (Pages F. et al. (2018) Lancet 391 (10135):2128-2139). Cold tumors are defined as having a low degree of immune cell infiltration, i.e. preferably have a low immunoscore. Hot tumors are defined as having an intermediate or high degree of immune cell infiltration, i.e. preferably have an intermediate or high immunoscore.

Some tumor types belong to the type of hot tumors even before treatment, for example melanoma. Nevertheless, the present invention enables a further enhanced activation of T cells and infiltration of immune cells into the tumor.

Cold tumors usually do not respond well to immunotherapies and cell-based therapies. The present invention is based on the discovery that an oncolytic virus can improve such responsiveness by increasing the infiltration of immune cells into the tumor and thereby positively influencing the TME. Patients with cold tumors (e.g. colorectal carcinoma, ovarian cancer, lung cancer), therefore especially benefit from the treatment. The tumor may as a result show better responsiveness to cell based therapies. Cold tumors may thereby be converted into hot tumors through the application of the parvovirus H-1 which functions as a "door-opener" and makes the tumor susceptible for the T cell therapy.

Thus, provided herein are immunotherapies, such as adaptive cell therapy, e.g., T cell therapy, in combination with parvovirus H-1 for treating subjects with cancer, particularly with a solid tumor. The T cell therapy includes cells that express recombinant receptors such as chimeric antigen receptors (CARs).

### Chimeric Antigen Receptors and CAR cells

"Chimeric Antigen Receptors" (CARs) are recombinant receptors that provide both antigen-binding and immune cell activating functions. CAR structure and engineering is reviewed, for example, in Dotti et al., Immunol Rev (2014) 257(1).

Thus, the Chimeric Antigen Receptor (CAR) refers to a recombinant polypeptide construct comprising at least an extracellular antigen binding domain, a transmembrane domain and a cytoplasmic signaling domain (also referred to herein as "an intracellular signaling domain") comprising a functional signaling domain derived from a stimulatory molecule as defined below. In some embodiments, the domains in the CAR polypeptide construct are in the same polypeptide chain, e.g., comprise a chimeric fusion protein. In some embodiments, the domains in the CAR polypeptide construct are not contiguous with each other, e.g., are in different polypeptide chains.

In some embodiments, the cytoplasmic signaling domain comprises a primary signaling domain (e.g., a primary signaling domain of CD3-zeta). In some embodiments, the cytoplasmic signaling domain further comprises one or more functional signaling domains derived from at least one costimulatory molecule as defined below. In some embodiments, the costimulatory molecule is chosen from 41BB (i.e., CD137), CD27, ICOS, and/or CD28. In some embodiments, the CAR comprises a chimeric fusion protein comprising an extracellular antigen recognition domain, a transmembrane domain and an intracellular signaling domain comprising a functional signaling domain derived from a stimulatory molecule. In some embodiments, the CAR comprises a chimeric fusion protein comprising an extracellular antigen recognition domain, a transmembrane domain and an intracellular signaling domain comprising a functional signaling domain derived from a co-stimulatory molecule and a functional signaling domain derived from a stimulatory molecule. In some embodiments, the CAR comprises a chimeric fusion protein comprising an extracellular antigen recognition domain, a transmembrane domain and an intracellular signaling domain comprising two functional signaling domains derived from one or more co- stimulatory molecule(s) and a functional signaling domain derived from a stimulatory molecule. In some embodiments, the CAR comprises a chimeric fusion protein comprising an extracellular antigen recognition domain, a transmembrane domain and an intracellular signaling domain comprising at least two functional signaling domains derived from one or more co-stimulatory molecule(s) and a functional signaling domain derived from a stimulatory molecule. In some embodiments the CAR comprises an optional leader sequence at the amino-terminus (N-ter) of the CAR fusion protein. In some embodiments, the CAR further comprises a leader sequence at the N-terminus of the extracellular antigen recognition domain, wherein the leader sequence is optionally cleaved from the antigen recognition domain (e.g., an scFv) during cellular processing and localization of the CAR to the cellular membrane. In this regard reference is made to Fig. 3.

A CAR that comprises an antigen binding domain (e.g., an scFv, a single domain antibody, or TCR (e.g., a TCR alpha binding domain or TCR beta binding domain)) that targets a specific cancer cell antigen or tumor marker X, wherein X can be a cancer cell antigen as described herein. For example, a CAR that comprises an antigen binding domain that targets CEA is referred to as CEA-CAR. The CAR can be expressed in any cell, e.g., an immune effector cell as described below (e.g., a T cell or an NK cell).

The present disclosure also provides a cell comprising or expressing a CAR according to the present disclosure. Also provided is a cell comprising or expressing a nucleic acid encoding a CAR according to the disclosure.

The cell may be an immune cell. The cell may be a cell of hematopoietic origin, e.g. a neutrophil, eosinophil, basophil, dendritic cell, lymphocyte, or monocyte. The lymphocyte may be e.g. a T cell, B cell, NK cell, NKT cell or innate lymphoid cell (ILC), or a precursor thereof. The cell may express e.g. CD3 polypeptides (e.g. CD3y CD3 or CD35), TCR polypeptides (TCRa or TCR), CD27, CD28, CD4 or CD8.

In a preferred embodiment, the cell is a T cell. In some embodiments, the T cell is a CD3+ T cell. In some embodiments, the T cell is a CD3+, CD8+ T cell. In some embodiments, the T cell is a cytotoxic T cell (e.g. a cytotoxic T lymphocyte (CTL)).

The use of CAR T-cells is associated with the advantage that they can be systemically administered, and will apply to both primary and metastasized tumors.

In some embodiments, the cell is an antigen-specific T cell. In embodiments herein, an "antigen-specific" T cell is a cell which displays certain functional properties of a T cell in response to the antigen for which the T cell is specific, or a cell expressing said antigen. In some embodiments, the properties are functional properties associated with effector T cells, e.g. cytotoxic T cells.

In some embodiments, an antigen-specific T cell may display one or more of the following properties: cytotoxicity, e.g. to a cell comprising/expressing antigen for which the T cell is specific; proliferation, IFNy expression, CD107a expression, IL-2 expression, TNFa expression, perforin expression, granzyme expression, granulysin expression, and/or FAS ligand (FASL) expression, e.g. in response to antigen for which the T cell is specific or a cell comprising/expressing antigen for which the T cell is specific. Antigen- specific T cells comprise a TCR capable of recognizing a peptide of the antigen for which the T cell is specific when presented by the appropriate MHC molecule. Antigen-specific T cells may be CD4+ T cells and/or CD8+ T cells.

Engineering of CARs into T cells may be performed during culture, in vitro, for transduction and expansion, such as happens during expansion of T cells for adoptive T cell therapy. Methods for collecting and engineering immune cells to express CARs are known to the skilled person and are described e.g. in Wang and Riviere Mol Ther. Oncolytics. (2016) 3: 16015. It will be appreciated that "at least one cell" encompasses plural cells, e.g. populations of such cells.

The cell comprising or expressing a CAR according to the present disclosure may be a eukaryotic immune cell as defined above, e.g. a mammalian immune cell. The mammal may be a human, or a non-human mammal (e.g. rabbit, guinea pig, rat, mouse or other rodent (including any animal in the order Rodentia), cat, dog, pig, sheep, goat, cattle (including cows, e.g. dairy cows, or any animal in the order Bos), horse (including any animal in the order Equidae), donkey, and non-human primate). In some embodiments, the cell may be from, or may have been obtained from, a human subject. Where the CAR-expressing cell is to be used in the treatment of a subject, the cell may be from the subject to be treated with the CAR-expressing cell (i.e. the cell may be autologous), or the cell may be from a different subject (i.e. the cell may be allogeneic).

Currently, most of the CAR-T clinical trials use autologous CAR-T, but the patient's own T cells usually have quality and quantity defects; and the production cost of autologous CAR-T is more expensive. Thus, also allogenic CAR-T may be useful. However, the antigen receptor TCR on allogeneic T cells may recognize allogeneic antigens in the recipient, thereby causing graft-versus-host disease (GVHD). In addition, the expression of HLA on allogeneic T cells can quickly cause host immune cell rejection reaction. Therefore, using gene editing tools such as ZFNs, TALENs and CRISPR/Cas9 to knock out TCR, MHC and related signaling pathway genes on allogeneic T cells to prevent host rejection of allogeneic CAR-T is the key step for the realization of universal CAR-T.

Optionally, the subject to be treated with CAR-T cells has undergone lymphodepletion. Myeloablative lymphodepletion may be achieved through thymectomy and/or irradiation. Nonmyeloablative lymphodepleting may be achieved through treatment with cyclophosphamide and fludarabine. The reason for the optional lymphodepletion is the reduction of the subject's lymphocyte pool prior to adoptive transfer of the CAR T cells. This may enhance treatment efficacy by eliminating regulatory T cells and competing elements of the subject's immune system ("cytokine sinks").

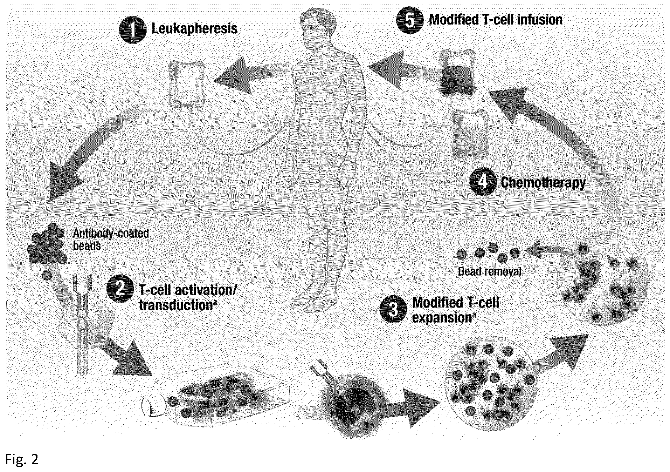

The principle of CAR-T therapy is shown in Fig. 2. T cells of patients are collected (e.g. by leukapheresis) and expanded and are modified to express chimeric antigen receptors (CAR) that recognize a single tumor antigen through genetic engineering. After a large number of CAR-T cells are expanded in-vitro they are returned to the patient for cellular immunotherapy. CAR, as a chimeric protein expressed by genes, contains the antigen-binding domain of an antibody (such as a single-chain antibody scFv) connected to the T cell signaling domain. The significant advantage of CAR-T cell adoptive immunity lies that cellular immunotherapy is more precise. The CAR-T cell adoptive immunotherapy system uses genetic modification of the T cells, and uses the principle of antigen-antibody binding to circumvent the MHC-restricted antigen presentation, thereby achieving precise targeting.

At present, the research and development of CAR-T therapy is mainly focused on the construction of CAR, through various modifications to enhance the targeting, immune killing, durability and safety of CAR-T cells.

In some embodiments the method steps for production of the at least one cell comprising a chimeric antigen receptor (CAR) specific for a cancer cell antigen may comprise one or more of: taking a blood or cancer biopsy sample from a subject; testing whether the sample expresses a specific cancer cell antigen, isolating and/or expanding at least one cell from the sample; culturing the at least one cell in in-vitro or ex-vivo cell culture; introducing into the at least one cell a CAR as described herein, or a nucleic acid encoding a CAR as described herein, thereby modifying the at least one cell; expanding the at least one modified cell; collecting the at least one modified cell; mixing the modified cell with an adjuvant, diluent, or carrier; administering the modified cell to the subject.

In some embodiments, the methods may additionally comprise treating the cell to induce/enhance expression of the CAR or nucleic acid encoding the CAR. For example, the nucleic acid may comprise a control element for inducible upregulation of expression of the CAR from the nucleic acid in response to treatment with a particular agent. In some embodiments, treatment may be in vivo by administration of the agent to a subject having been administered with a modified cell according to the disclosure. In some embodiments, treatment may be ex vivo or in vitro by administration of the agent to cells in culture ex vivo or in vitro.

The skilled person is able to determine appropriate reagents and procedures for adoptive transfer of cells according to the present disclosure, for example by reference to Dai et al., 2016 J Nat Cancer Inst 108(7): 439.

In the process of CAR protein expression on the surface of T cells, viral vectors are needed to synthesize DNA sequences that can express CAR proteins in cells through DNA synthesis technology. Thus, the CAR DNA sequences are loaded into plasmid vectors through molecular cloning technology. Preferably, plasmid vectors express genes or DNA sequences at multiple cloning sites into proteins in cells. After these CAR proteins are expressed, they are anchored in the T cells. surface. In the alternative, virus-mediated gene expression technologies may be used, such as lentivirus, retrovirus, adenovirus, adeno-associated virus (AAV), etc.

Cancer cell antigens (also called "tumor antigens") that are suitable for cancer treatment by CAR-T technology are reviewed by Zarour HM, DeLeo A, Finn OJ, et al. Categories of Tumor Antigens. In: Kufe DW, Pollock RE, Weichselbaum RR, et al., editors. Holland-Frei Cancer Medicine. 6th edition. Hamilton (ON): BC Decker; 2003, wherein for the present invention reference is made to these antigens and they are incorporated herein by reference. Examples of cancer cell antigens include without limitation: CD19; CD123; CD22; CD30; CD70, CD97, CD171; CS-1; C-type lectin-like molecule- 1, CD33; epidermal growth factor receptor variant III (EGFRvIII); ganglioside G2 (GD2); ganglioside GD3; TNF receptor family member; B-cell maturation antigen; Tn antigen ((Tn Ag) or (GaINAca-Ser/Thr)); prostate- specific membrane antigen (PSMA); Receptor tyrosine kinase-like orphan receptor 1 (ROR1); Fms-Fike Tyrosine Kinase 3 (FFT3); Tumor-associated glycoprotein 72 (TAG72); CD38; CD44v6; Carcinoembryonic antigen (CEA); Cancer antigen 125 (CA125), Epithelial cell adhesion molecule (EPCAM); B7H3 (CD276); KIT (CD117); Interleukin-13 receptor subunit alpha-2; Mesothelin; Interleukin 11 receptor alpha (IL-IIRa); prostate stem cell antigen (PSCA); Protease Serine 21; vascular endothelial growth factor receptor 2 (VEGFR2); Lewis(Y) antigen; CD24; Platelet-derived growth factor receptor beta (PDGFR-beta); Stage- specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); prostatic acid phosphatase (PAP); elongation factor 2 mutated (EFF2M); Ephrin B2; fibroblast activation protein alpha (FAP); insulin-like growth factor 1 receptor (IGF-I receptor), carbonic anhydrase IX (CAIX); Proteasome (Prosome, Macropain) Subunit, Beta Type, 9 (LMP2); glycoprotein 100 (gplOO); oncogene polypeptide consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3; transglutaminase 5 (TGS5); high molecular weight - melanoma-associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein- coupled receptor class C group 5, member D (GPRC5D); chromosome X open reading frame 61 (CXORF61); CD97; CD179a; anaplastic lymphoma kinase (ALK); Polysialic acid; placenta- specific 1 (PLAC1); hexasaccharide portion of globoH glycoceramide (GloboH); mammary gland differentiation antigen (NY-BR-1); uroplakin 2 (UPK2); Hepatitis A vims cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma- associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; survivin; telomerase; prostate carcinoma tumor antigen-1, RAS family antigen or mutant (k-RAS, N-RAS), human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); prostate specific antigen (PSA), Androgen receptor; Cyclin BI; v-myc avian myelocy tomato sis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-like, Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (FCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); cyclin-dependent kinase inhibitor p16^{INK4a}; intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); MSI frameshift mutants (e.g. those mentioned in WO 2014/090265 A1), Fc receptor-like 5 (FCRL5); or immunoglobulin lambda- like polypeptide 1 (IGLL1).

In some embodiments, the antigen is selected from mesothelin, EGFRvIII, GD2, Tn antigen, PSMA, PSA, CD70, CD97, TAG72, CD44v6, CEA, CA125, EPCAM, KIT, IL-13Ra2, leguman, GD3, CD171, IL-I IRa, PSCA, MAD-CT- 1, MAD-CT-2, VEGFR2, LewisY, CD24, PDGFR-beta, SSEA-4, folate receptor alpha, ERBBs (e.g., ERBB2), Her2/neu, MUC1, EGFR, NCAM, Ephrin B2, CAIX, LMP2, sLe, HMWMAA, o-acetyl-GD2, folate receptor beta, TEM1/CD248, TEM7R, FAP, Legumain, HPV E6 or E7, p16^{INK4a}, ML-IAP, CLDN6, TSHR, GPRC5D, ALK, polysialic acid, Fos-related antigen, neutrophil elastase, TRP-2, CYP1B1, sperm protein 17, beta human chorionic gonadotropin, AFP, thyroglobulin, PLAC1, globoH, RAGE1, MN-CA IX, MSI frameshift mutants, human telomerase reverse transcriptase, intestinal carboxyl esterase, mut hsp 70-2, NA-17, NY-BR-1, UPK2, HAVCR1, ADRB3, PANX3, NY-ESO-1, GPR20, Ly6k, OR51E2, TARP, or GFRa4.

Examples for CAR-T therapy and how to produce and prepare the CARs and T cells are described in Katz et al., Cancer Gene Therapy (2020), 27:341-355, Hege et al., Journal for ImmunoTherapy of Cancer (2017), 5:22 and Koneru et al., Journal of Translational Medicine (2015), 13:102. These methods are incorporated herein by reference.

### Oncolytic virus

The term "oncolytic virus" means a virus from the Parvoviridae family and means particularly a "parvovirus", more particularly parvovirus H-1 or a related rodent parvovirus selected from Lulll, Mouse minute virus (MMV), Mouse parvovirus (MPV), Rat minute virus (RMV), Rat parvovirus (RPV), or Rat virus (RV).

As used herein the oncolytic virus comprises wild-type or modified replication-competent derivatives thereof, as well as related viruses or vectors based on such viruses or derivatives. Suitable oncolytic viruses, derivatives, etc. as well as cells which can be used for actively producing said viruses and which are useful for therapy, are readily determinable within the skill of the art based on the disclosure herein, without undue empirical effort.

Parvovirus H-1 (H-1PV) belongs to the *Parvoviridae* family and is a small (~25 nm in diameter), non-enveloped icosahedral particle containing a 5.1 kb long single-stranded DNA genome. The genomic organization of H-1 PV consists of two transcriptional units under the control of two promoters, the P4 early promoter and P38 late promoter. P4 regulates the expression of the gene encoding for the non-structural (NS) proteins (NS1 and NS2) and the P38 the one encoding for the capsid (VP) proteins (VP1, VP2, VP3). The virus multiplies preferentially in fast dividing cancer cells. This onco-selectivity is not based on a better uptake of the virus by cancerous cells, but rather is due to the fact that cancer cells overexpress factors such as cyclin A, E2F, or CREB/ATF required for virus DNA replication. Furthermore, cancer cells are often defective in their ability to mount an efficient antiviral immune response favouring viral multiplication. The virus is known to activate multiple cell death pathways. Depending on cell type and growing conditions, H-1PV may induce apoptosis, necrosis, or cathepsin B-dependent cell death. The major non-structural protein NS1 is the master regulator of virus DNA replication, viral gene expression and cytotoxicity. The sole expression of NS1, similarly to the entire virus, is sufficient to induce cell cycle arrest, apoptosis and cell lysis via accumulation of reactive oxygen species and DNA damage.

### Treatment Details

Preferably, in the medical preparation of the present invention the oncolytic virus, i.e. parvovirus H-1, and the immune cells (e.g. T cells) genetically modified to express a chimeric antigen receptor (CAR) are present in an effective dose and combined with a pharmaceutically acceptable carrier.

According to the present invention, the terms "pharmaceutical combination", "pharmaceutical composition" or "medical preparation" are used interchangeably.

The terms "individual" and "subject" are used herein interchangeably. They refer to a human or another mammal (e.g. mouse, rat, rabbit, dog, cat, cattle, swine, sheep, horse or primate) that can be afflicted with or is susceptible to a disease or disorder (e.g., cancer) but may or may not have the disease or disorder. In many embodiments, the individual is a human being. Unless otherwise stated, the terms "individual" and "subject" do not denote a particular age, and thus encompass adults, elderlies, children, and newborns. In embodiments of the present disclosure, the "individual" or "subject" is a "patient". The term "patient" means an individual or subject for treatment, in particular a diseased individual or subject.

In one embodiment of the disclosure, the aim is to provide an immune response against diseased cells expressing an antigen such as cancer cells expressing a tumor antigen, and to treat a disease such as a cancer disease involving cells expressing an antigen such as a tumor antigen. An immune response against an antigen may be elicited which may be therapeutic or partially or fully protective. Pharmaceutical compositions described herein are applicable for inducing or enhancing an immune response.

Pharmaceutical compositions described herein are thus useful in a prophylactic and/or therapeutic treatment of a disease involving an antigen.

As used herein, "immune response" refers to an integrated bodily response to an antigen or a cell expressing an antigen and refers to a cellular immune response and/or a humoral immune response. A cellular immune response includes, without limitation, a cellular response directed to cells expressing an antigen. Such cells may be characterized by expression of an antigen on their cell surface or by presentation of an antigen with class I or class II MHC molecule. The cellular response relates to T lymphocytes, which may be classified as helper T cells (also termed CD4+ T cells) that play a central role by regulating the immune response or killer cells (also termed cytotoxic T cells, CD8+ T cells, or CTLs) that induce apoptosis in infected cells or cancer cells. In one embodiment, administering a pharmaceutical composition of the present disclosure involves stimulation of an anti-tumor CD8+ T cell response against cancer cells expressing one or more tumor antigens.

The present disclosure contemplates an immune response that may be protective, preventive, prophylactic and/or therapeutic. As used herein, "induces [or inducing] an immune response" may indicate that no immune response against a particular antigen was present before induction or it may indicate that there was a basal level of immune response against a particular antigen before induction, which was enhanced after induction. Therefore, "induces [or inducing] an immune response" includes "enhances [or enhancing] an immune response".

The term "immunotherapy" relates to the treatment of a disease or condition by inducing, or enhancing an immune response.

The term "vaccination" or "immunization" describes the process of administering an antigen to an individual with the purpose of inducing an immune response, for example, for therapeutic or prophylactic reasons.

As used herein the term "agent" is understood to mean a substance that produces a desired effect in a tissue, system, animal, mammal, human, or other subject.

By the term "treating" and derivates thereof as used herein, is meant therapeutic therapy. In reference to a particular condition, treating means: (1) to ameliorate the condition or one or more of the biological manifestations of the conditions, (2) to interfere with (a) one or more points in the biological cascade that leads to or is responsible for the condition or (b) one or more of the biological manifestations of the condition (3) to alleviate one or more of the symptoms, effects or side effects associated with the condition, or (4) to slow the progression of the condition or one or more of the biological manifestations of the condition.

The term "pharmaceutically effective amount" or "therapeutically effective amount" refers to the amount which achieves a desired reaction or a desired effect alone or together with further doses. In the case of the treatment of a particular disease, the desired reaction preferably relates to inhibition of the course of the disease. This comprises slowing down the progress of the disease and, in particular, interrupting or reversing the progress of the disease. The desired reaction in a treatment of a disease may also be delay of the onset or a prevention of the onset of said disease or said condition. An effective amount of the compositions described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the compositions described herein may depend on various of such parameters. In the case that a reaction in a patient is insufficient with an initial dose, higher doses (or effectively higher doses achieved by a different, more localized route of administration) may be used. As used herein, "effective amount" means that amount of any of the ingredients or components of the medical preparation that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder or side effect. The term also includes within its scope amounts effective to enhance normal physiological function. An "effective dose" useful for treating and/or preventing these diseases or disorders may be determined using methods known to one skilled in the art.

The administration of a therapeutically effective amount of the combinations of the invention are advantageous over the individual component compounds in that the combinations provide one or more of the following improved properties when compared to the individual administration of a therapeutically effective amount of a component compound: i) a greater anticancer effect than the most active single agent, ii) synergistic or highly synergistic anticancer activity, iii) a dosing protocol that provides enhanced anticancer activity with reduced side effect profile, iv) a reduction in the toxic effect, profile, v) an increase in the therapeutics window, or vi) an increase in the bioavailability of one or both of the component compounds.

"Pharmaceutically acceptable" is meant to encompass any carrier, which does not interfere with the effectiveness of the biological activity of the active ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at an effective dose. Additional pharmaceutically compatible carriers can include gels, bioadsorbable matrix materials, implantation elements containing the therapeutic agent, or any other suitable vehicle, delivery or dispensing means or material(s).

The pharmaceutical compositions of the present invention may contain salts, buffers, preservatives, and optionally other therapeutic agents. In one embodiment, the pharmaceutical compositions of the present disclosure comprise one or more pharmaceutically acceptable carriers, diluents and/or excipients.

Suitable preservatives for use in the pharmaceutical compositions of the present disclosure include, without limitation, benzalkonium chloride, chlorobutanol, paraben and thimerosal.

The term "excipient" as used herein refers to a substance which may be present in a pharmaceutical composition of the present disclosure but is not an active ingredient. Examples of excipients, include without limitation, carriers, binders, diluents, lubricants, thickeners, surface active agents, preservatives, stabilizers, emulsifiers, buffers, flavoring agents, or colorants.

The term "diluent" relates a diluting and/or thinning agent. Moreover, the term "diluent" includes any one or more of fluid, liquid or solid suspension and/or mixing media. Examples of suitable diluents include ethanol, glycerol and water.

The term "carrier" refers to a component which may be natural, synthetic, organic, inorganic in which the active component is combined in order to facilitate, enhance or enable administration of the pharmaceutical composition. A carrier as used herein may be one or more compatible solid or liquid fillers, diluents or encapsulating substances, which are suitable for administration to subject. Suitable carrier include, without limitation, sterile water, Ringer, Ringer lactate, sterile sodium chloride solution, isotonic saline, polyalkylene glycols, hydrogenated naphthalenes and, in particular, biocompatible lactide polymers, lactide/glycolide copolymers or polyoxyethylene/polyoxy-propylene copolymers. In one embodiment, the pharmaceutical composition of the present disclosure includes isotonic saline.

Pharmaceutically acceptable carriers, excipients or diluents for therapeutic use are well known in the pharmaceutical art, and are described, for example, in Remington's Pharmaceutical Sciences, Mack Publishing Co. (A. R Gennaro edit. 1985).

Pharmaceutical carriers, excipients or diluents can be selected with regard to the intended route of administration and standard pharmaceutical practice.

As used herein the term "cancer" refers to an abnormal growth of cells or tissue and is understood to include malignant neoplastic growths. The term "neoplastic" means of or related to a neoplasm. In some embodiments the cancer is a solid tumor, particularly liver cancer (e.g. hepatocellular carcinoma), gastric cancer, ovarian cancer, endometrial cancer, cervical cancer, colorectal cancer (e.g. (adeno)carcinoma of cecum, appendix, colon ascendens, colon descendens, colon transversum, colon sigmoideum, rectum carcinoma or anus carcinoma), lung cancer (e.g. lung squamous cell carcinoma, non-small-cell lung cancer (NSCLS), small-cell lung cancer (SCLC)), soft-tissue sarcoma, osteosarcoma, fibrosarcoma, skin cancer (e.g. malignant melanoma), testis cancer, breast cancer, fibrosarcoma, neuroblastoma, brain cancer (e.g. gliomas: ependymomas, astrocytomas, oligodendrogliomas, brainstem glioma, oligoastrocytomas (e.g. glioblastoma multiforme, medulloblastoma)), bladder cancer, intestinal cancer, prostate cancer, kidney cancer (e.g. renal cell carcinoma), pancreatic cancer (e.g. pancreatic ductal adenocarcinoma), pleural mesothelioma, head and neck squamous cell carcinoma (HNSCC), nasopharyngeal carcinoma (NPC) or oropharyngeal carcinoma (OPC). The term "cancer" also encompasses metastases of the mentioned tumors in various organs. In a further preferred embodiment the tumour to be treated are recurrent tumours. A particular advantage of the medical formulation of the present invention is that even cancer initiating stem cells can be successfully treated. This has a positive effect as regards the avoidance of the recurrence of the tumours and metastasis formation.

In some embodiments the cancer is a hematological cancer, particularly acute or chronic leukemia or a lymphoma. In some embodiments the leukemia is selected from acute myeloid leukemia (AML), acute lymphoid leukemia (ALL), chronic myeloid leukemia (CML), or chronic lymphoid leukemia (CLL). In some embodiments, the lymphoma is non-Hodgkin's lymphoma. In some embodiments, the non-Hodgkin's lymphoma is mantle cell lymphoma, follicular lymphoma, diffuse large B-cell lymphoma, marginal zone lymphoma or Burkitt's lymphoma.

Administration of the compounds may be achieved through different systemic or local ways, e.g. by intravenous, intraperitoneal, subcutaneous, intramuscular, topical, intratumoral, nasal or intradermal administration. The route of administration, of course, depends on the kind of therapy and the kind of compounds contained in the pharmaceutical composition. The dosage regimen of the virus and CAR-T is readily determinable within the skill of the art, by the attending physician based on patient data, observations and other clinical factors, including for example the patient's size, body surface area, age, sex, the particular virus, the particular inhibitor etc. to be administered, the time and route of administration, the tumor type and characteristics, general health of the patient, and other drug therapies to which the patient is being subjected. Selecting a dosage regimen (also referred to herein as an administration regimen) for a combination therapy of the invention depends on several factors, including the serum or tissue turnover rate of the entity, the level of symptoms, the immunogenicity of the entity, and the accessibility of the target cells, tissue or organ in the individual being treated. Preferably, a dosage regimen maximizes the amount of each therapeutic agent delivered to the patient consistent with an acceptable level of side effects. Accordingly, the dose amount and dosing frequency of each therapeutic agent in the combination depends in part on the particular therapeutic agent, the severity of the cancer being treated, and patient characteristics. Guidance is selecting appropriate doses of antibodies, cytokines, and small molecules are available. See, e.g., Wawrzynczak (1996) Antibody Therapy, Bios Scientific Pub. Ltd. Oxfordshire, UK; Kresina (ed.) (1991) Monoclonal Antibodies, Cytokines and Arthritis, Marcel Dekker, New York, NY; Bach (ed.)(1193) Monoclonal Antibodies and Peptide Therapy in Autoimmune Diseases, Marcek Dekker, New York, NY; Beart et al, (2003) New Engl. J. Med. 348:601-608; Milgrom et al. (1999) New Engl.J. Med 341:1966-1973; Slamon et al. (2001) New Engl. J. Med. 344:783-792; Beniaminovitz et al. (2000) New Engl. J. Med. 342:613-619; Ghosh et al. (2003) New Engl. J. Med. 348:24-32; Lipsky et al. (2000) New Engl. J. Med. 343; 1594-1602; Physicians'Desk Reference 2003 (Physicians'Desk Reference, 57th ed); Medical Economics Company; ISBN; 1563634457; 57th edition (November 2002). Determination of the appropriate dosage regimen may be made by the clinician, e.g., using parameters or factors known or suspected in the art to affect treatment or predicted to affect treatment, and will depend, for example, the patient's clinical history (e.g., previous therapy), the type and stage of the cancer to be treated and biomarkers of response to one or more of the therapeutic agents in the combination therapy.

Since the virus in the combination with the CAR cells according to the invention comprises infectious virus particles with the ability to penetrate through the blood system, treatment can be performed or at least initiated by intravenous injection of the virus. Since long-term intravenous treatment may be susceptible to becoming inefficient as a result of the formation of neutralizing antibodies to the virus, different modes of administration can be adopted after an initial regimen intravenous viral administration, or such different administration techniques, e.g., intratumoral virus administration, can be alternatively used throughout the entire course of viral treatment. In a preferred embodiment, however, the administration occurs throughout the whole course of treatment by intravenous administration.

As another specific administration technique, the virus (virus, vector and/or cell agent) can be administered to the patient from a source implanted in the patient. For example, a catheter, e.g., of silicone or other biocompatible material, can be connected to a small subcutaneous reservoir (Rickham reservoir) installed in the patient during tumor removal or by a separate procedure, to permit the parvovirus composition to be injected locally at various times without further surgical intervention. The virus or derived vectors can also be injected into the tumor by stereotactic surgical techniques or by navigation targeting techniques.

Administration of the virus can also be performed by continuous infusion of viral particles or fluids containing viral particles through implanted catheters at low flow rates using suitable pump systems, e.g., peristaltic infusion pumps or convection enhanced delivery (CED) pumps.

A yet another method of administration of the viral combination part is from an implanted article constructed and arranged to dispense the parvovirus to the desired cancer tissue. For example, wafers can be employed that have been impregnated with the virus, particularly parvovirus H-1, wherein the wafer is attached to the edges of the resection cavity at the conclusion of surgical tumour removal. Multiple wafers can be employed in such therapeutic intervention. Cells that actively produce the virus, or virus-based vectors, can be injected into the tumour or into the tumoral cavity after tumour removal.

The CAR T-cells and CAR-T cell compositions can be administered according to a suitable dosage schedule. In certain embodiments, the CAR T-cells are administered once, with subsequent doses depending on clinical criteria. If an individual does not respond or only partially responds said patient can have a CAR-T cell composition administered a second, third, or fourth time until the desired clinical response is observed. A dosage of CAR-T cells will generally comprise at least 1x10⁶ cells, but no more than 5x10⁸ cells. Cells can be administered based upon a total amount of an individual's viable PBMC that were transduced with a CAR construct. In certain embodiments, a single dosage comprises 1 million transduced PBMCs to 100 million transduced PBMCs.

The parvovirus can be administered according to a suitable dosage schedule. In certain embodiments, the virus is administered once, with subsequent doses depending on clinical criteria. If an individual does not respond or only partially responds said patient can have a second, third, or fourth administration until the desired clinical response is observed. A dosage of the parvovirus will generally comprise at least 1x10⁶ pfu, but no more than 5x10¹¹ pfu. Preferably dosages between 1x10⁷ pfu and 5x10¹⁰ pfu are administered.

It can also allow the clinical use of the virus and/or CAR cells at lower therapeutic doses preserving or even enhancing anticancer efficacy while increasing safety and reducing and/or avoiding side effects. In view of the strong synergistic effect between the virus and the CAR cells it is possible to foresee the reduction of the therapeutic doses, e.g. half or a third of the previously used single component doses are preserving the desired therapeutic effect. In view of the reduced doses (severe) side effects may be reduced or even avoided.

In case of parvovirus the infection effects kill tumour cells but does not harm normal cells and such infection can, for example, be carried out by intravenous or intratumoural use of a suitable parvovirus, e.g., parvovirus H-1, or a related virus or vectors based on such viruses, to effect tumour-specific therapy without adverse neurological or other side effects.

A combination therapy of the invention is typically used to treat a tumor that is large enough to be found by palpation or by imaging techniques well known in the art, such as MRI, ultrasound, or CAT scan. In some preferred embodiments, a combination therapy of the invention is used to treat an advanced stage tumor having dimensions of at least about 200 mm³, 300mm³, 400mm³, 500mm³, 750mm³, or up to 1000mm³.

A combination therapy of the invention may be used prior to or following surgery to remove a tumor and may be used prior to, during or after a chemotherapy or radiation therapy.

Thus, in some embodiments, the treatment may further comprise other therapeutic or prophylactic intervention, e.g. chemotherapy, immunotherapy, radiotherapy, surgery, vaccination and/or hormone therapy. Such other therapeutic or prophylactic intervention may occur before, during and/or after the therapies encompassed by the disclosure, and the deliveries of the other therapeutic or prophylactic interventions may occur via different administration routes as the therapies of the disclosure. Chemotherapy and radiotherapy respectively refer to treatment of a cancer with a therapeutic agent or with ionising radiation (e.g. radiotherapy using X-rays or g-rays).

In some embodiments, at least one of the therapeutic agents in the combination therapy is administered using the same dosage regimen (dose, frequency and duration of treatment) that is typically employed when the agent is used as monotherapy for treating the same cancer. In other embodiments, the patient receives a lower total amount of at least one of the therapeutic agents in the combination therapy than when the agent is used as monotherapy, e.g., smaller doses, less frequent doses, and/or shorter treatment duration.

The additional therapeutic agent may be, e.g., a chemotherapeutic agent, a biotherapeutic agent (including but not limited to antibodies, e.g. to VEGF, EGFR, Her2/neu; growth factor receptors, CD20, CD40, CD40L, CTLA-4, OX-40 4-1BB, and ICOS; antibody fragments), nucleic acid (DNA or RNA), an immunogenic agent (for example, attenuated cancerous cells, tumor antigens, antigen presenting cells such as dendritic cells pulsed with tumor derived antigen or nucleic acids), immune stimulating cytokines (for example, IL-2, IFN-α, IFN-γ, GM-CSF), and cells transfected with genes encoding immune stimulating cytokines (such as but not limited to GM-CSF) or checkpoint inhibitors (such as but not limited to anti-PD1 or anti-PD-L1 antibodies, e.g. nivolizumab, pembrolizumab, ipilimumab, tremelimumab, avelumab).

Examples of chemotherapeutic agents include alkylating agents such as cyclosphosphamide, busulfan, a camptothecin, chlorozotocin, fotemustine, lomustine, nimustine, ranimustine, antibiotics, bleomycins, caminomycin, dactinomycin, daunorubicin, idarubicin, 5-flourouracil (5-FU), methotrexate, cytarabine, platinum analog such as cisplatin and carboplatin; vinblastine, platinum; etoposide (VP-16); ifosfamide, mitoxantrone; vincristine; vinorelbine; novantrone; teniposide; edatrexate; daunomycin; aminopterin, xeloda; ibandronate; topoisomerase inhibitors; difluoromethylornithine (DMFO); retinoids, tamoxifen, raloxifene, droloxifene, 4-hydroxytamoxifen, trioxifene, keoxifene or aromatase inhibitors.

The present invention also relates to the use of (a) an oncolytic virus (particularly parvovirus H-1) and (b) immune cells (particularly T cells) genetically modified to express a chimeric antigen receptor (CAR) for the preparation of (a) therapeutic preparation(s) or pharmaceutical composition(s) or combination for the treatment of cancer.

The mode of administration of (a) and (b) may be simultaneously or sequentially, wherein it is preferred to administer (a) and (b) sequentially or separately. This means that (a) and (b) may be provided in a single unit dosage form for being taken together or as separate entities (e.g. in separate containers) to be administered simultaneously or with a certain time difference. This time difference may be between 1 hour and 1 week, preferably between 12 hours and 3 days, most preferred are 24 - 60 hours. In addition, it is possible to administer the virus via another administration way than the CAR cells. In this regard it may be advantageous to administer either the virus or CAR cells intratumourally and the other systemically. In a particular preferred embodiment the virus is administered intravenously and the CAR cells intratumourally. Preferably, the virus and the CAR cells are administered as separate compounds. Concomitant treatment with the two agents is also possible.

Each therapeutic agent in a combination therapy of the invention may be administered either alone or in a medicament (also referred to herein as "pharmaceutical composition" or "medical preparation") which comprises the therapeutic agent and one or more pharmaceutically acceptable carriers, excipients and diluents, in amounts according to standard pharmaceutical practice as mentioned above. Each therapeutic agent in a combination therapy of the invention may be administered simultaneously, concurrently or sequentially in any order.

Simultaneous administration refers to administration of the agents together, for example as a pharmaceutical composition containing the agents (i.e. a combined preparation), or immediately after each other and optionally via the same route of administration, e.g. to the same artery, vein or other blood vessel.

Sequential administration refers to administration of one or more of the agents followed after a given time interval by separate administration of another of the agents. Sequential administration is particularly useful when the therapeutic agents in the combination therapy are in different dosage forms (solid/liquid) and/or are administered on different dosing schedules, e.g. one is administered at least daily and a biotherapeutic that is administered less frequently, such as once weekly, once every two weeks, or once every three weeks. The time interval may be any time interval, including hours, days, weeks or months. In some embodiments sequential administration refers to administrations separated by a time interval of one of at least 10 min, 30 min, 1 hr, 6 hrs, 8 hrs, 12 hrs, 24 hrs, 36 hrs, 48 hrs, 3 days, 4 days, 5 days, 6 days, 1 week, 2 weeks, 3 weeks, 1 month, 6 weeks, 2 months, 3 months, 4 months, 5 months or 6 months. In a preferred embodiment the parvovirus is given first, i.e. with a certain time difference prior to the administration of the CAR-T cells. This time difference may be at least 10 hours but may be also 4 days, preferably it is 18-72 hours, most preferably between 24 and 60 hours..It is not required that the two agents are administered by the same route, although this is the case in some embodiments.

The CAR cells and oncolytic virus described herein may be provided as a kit which comprises a first container and a second container and a package insert. The first container contains at least one dose of CAR cells, the second container contains at least one dose of the oncolytic virus, and the package insert, or label, which comprises instructions for treating a patient for cancer using the therapeutic preparation. The first and second containers may be comprised of the same or different shape (e.g., vials, syringes and booles) and/or material (e.g. Plastic or glass). The kit may further comprise other materials that may be useful in administering the preparation, such as diluents, filters, IV bags and lines, needles and syringes.

In the present invention it has been shown for the first time that the combinatorial use of the oncolytic virus H-1PV and CAR cells may be a valid approach against solid tumors.

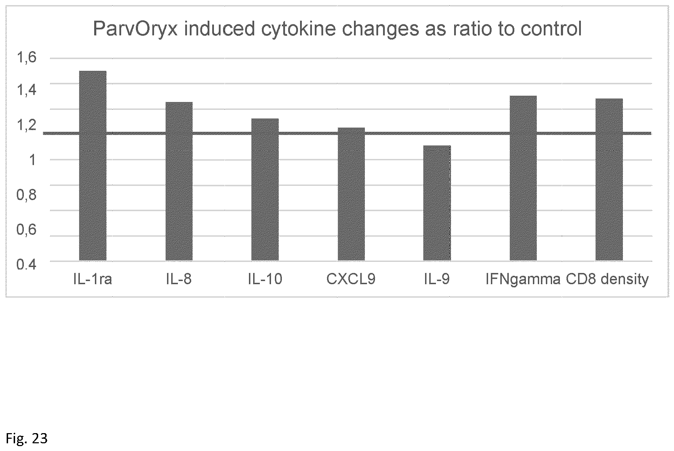

Without the intent of being bound to a theory, as previously mentioned tumors hide themselves from attacks by the immune system. This results in immune tolerance of the body to the tumor since many solid tumors have a microenvironment which makes it impossible that the activated immune cells invade into the tumor. This invasion into the tumor is now made possible by using the oncolytic virus, in particular a parvovirus, more particularly parvovirus H-1, which attacks the tumor and changes its microenvironment. In other words, the oncolytic virus is able to make the tumor "naked" through oncolysis and the CAR-T cells are able to start the invasion into the tumor. The oncolytic virus may be seen as a door opener for a successful immune response due to its ability to stimulate pro-inflammatory and pro-migratory cytokines and chemokines (Fig. 23). With this concept it should be possible to treat also solid tumors where CART-T treatment failed in the past since the oncolytic virus treatment transforms a non-immunogenic tumor in an immunogenic one. In view of the general principle this works with any oncolytic virus as long as it changes the tumor microenvironment and with any cell-based therapy. This could lead to long-term effects in prevention of disease relapse, potentially adding to initial oncolysis. The combination of effects renders the tumor more susceptible to the immune system, in particular after previous therapy with the virus.

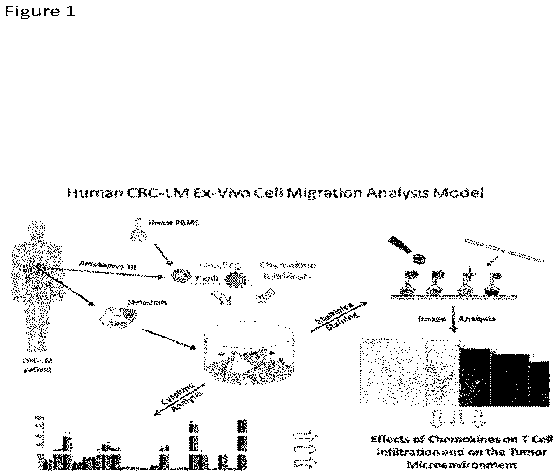

The invasive margin of tumors has been discovered as a highly specific region dominated by T cell attracting chemokines and myeloid-cell related factors accompanied by many different immune cell subtypes including immunosuppressive cells and T cells (Halama et al., Cancer Cell 29: 587-601 (2016); WO 2016/066634 A2). As such a distinct microenvironment is difficult to reproduce in animal models (Ellis and Fidler, Nat. Med. 16: 974-975 (2010)), the inventors established an ex vivo cell migration analysis model to study T cell infiltration and positioning in the original environment of tumor patients. The experimental design of the model is shown in Figure 1. The use of colorectal cancer liver metastases (CRC-LM) has to be understood as exemplary. The principle is the same for any tumor tissue.

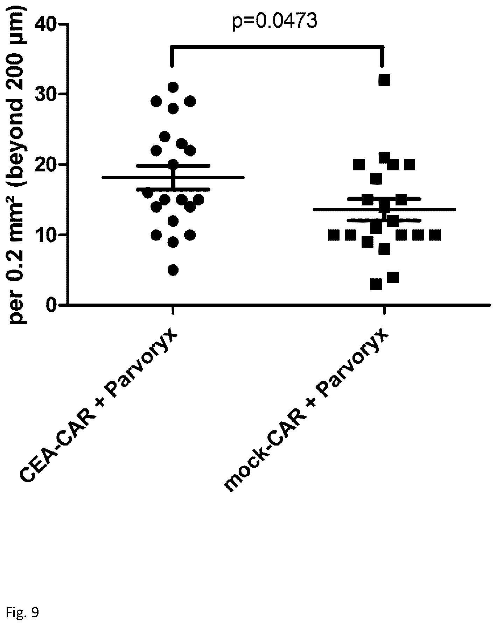

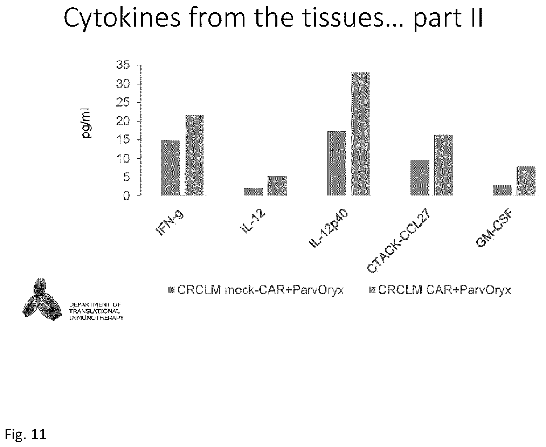

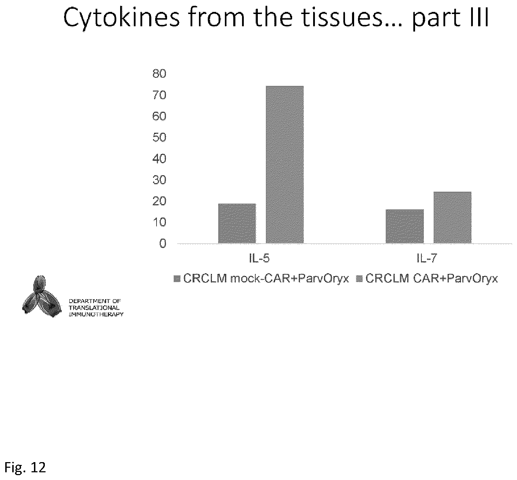

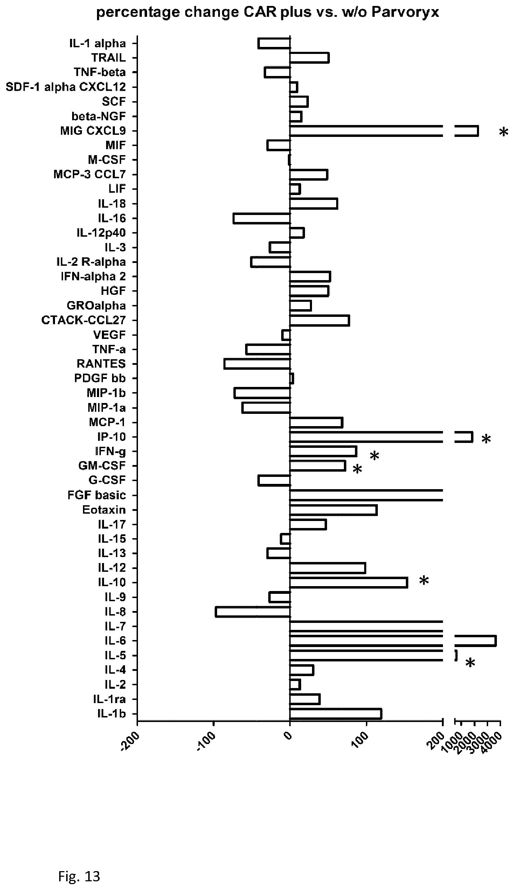

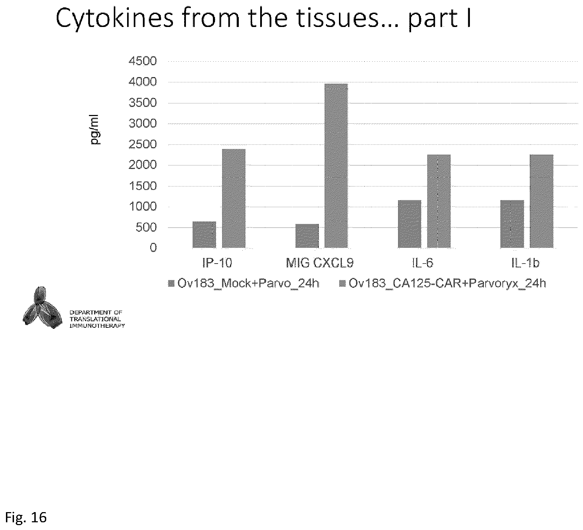

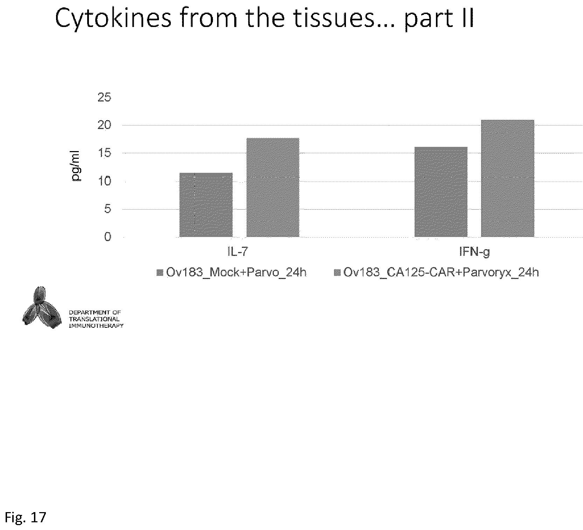

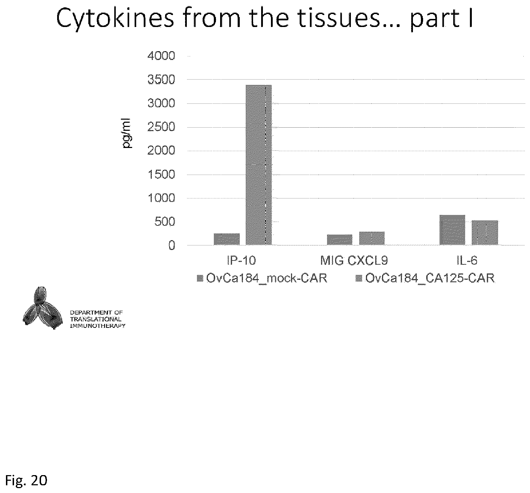

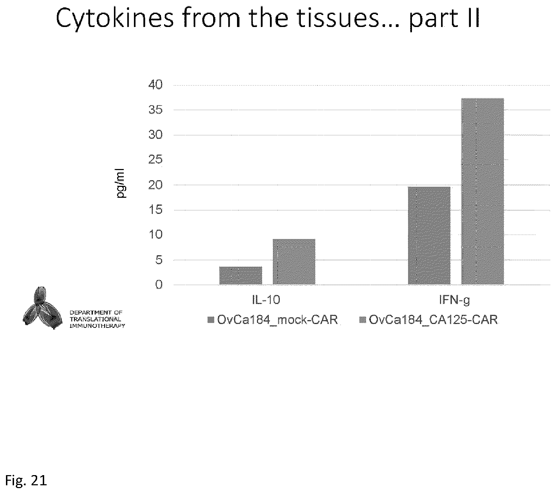

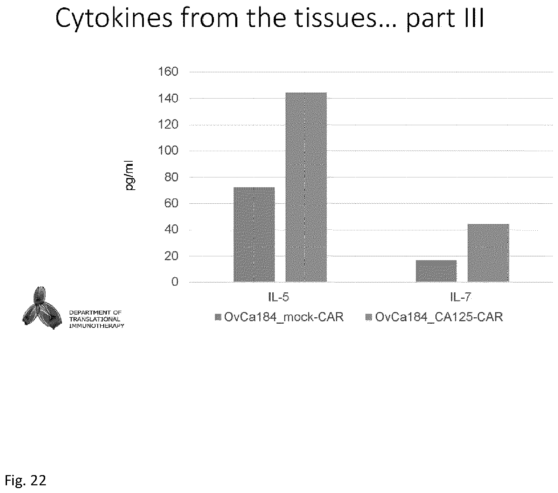

Thus, in the present application the proof-of-concept for the combinatorial effect has been made in an explant model (Fig. 1) which is able to reproduce the distinct microenvironment in the original environment of samples taken from tumor patients so that an ex vivo cell migration analysis to study T cell infiltration can be performed. In the present application it has been shown that the engaging CARs activate and modulate the tumor environment which can be seen from increased TH1 cytokines and more pro-migratory chemokines. In addition, upregulation of tonic T cell stimulatory interleukins (prolonged activation and differentiation initiated) has been seen. CARs carrying a cancer cell antigen that is associated with the tested tumor sample (e.g. CEA <-> colorectal cancer; CA-125 <-> ovarian carcinoma) produces dramatically different cytokine environment than compared with "mock-CARs" which carry a cancer cell antigen that is not associated with the tested tumor sample (c.f. Figs. 9, 10, 11, 12, 16, 17, 20, 21, 22). Thus, a distinct feature of CEA-CARs versus mock-CARs has been recognized, which means that specific CARs and their activation produce a distinct anti-tumoral environment. In the present application CEA-CARs were used as mock-CARs in the ovarian cancer model and CA-125 CARs were used as mock-CARs in the CRC-LM cancer model. When comparing the cytokine modulation between CARs with and without added parvovirus H-1, it has been clearly shown that added parvovirus results in T cell activation and enhanced migration but also antigen presentation, including GM-CSF, IL-10, CXCL9, CXCL10 (IP-10), IL-5 and IL-6 (Fig. 13). This means that a massive increase of TH1 type cytokines, a massive upregulation of pro-migratory chemokines and upregulation of tonic T cell stimulatory interleukines (prolonged activation and differentiation initiated) occurs.

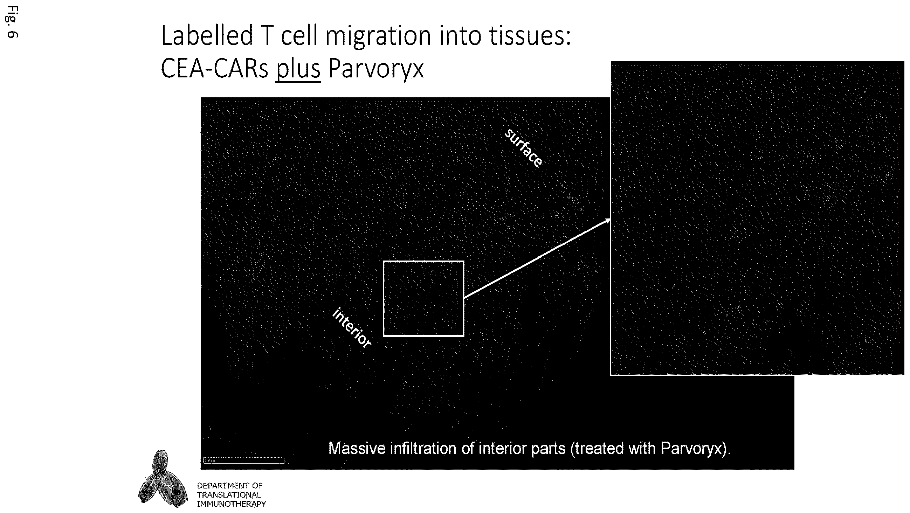

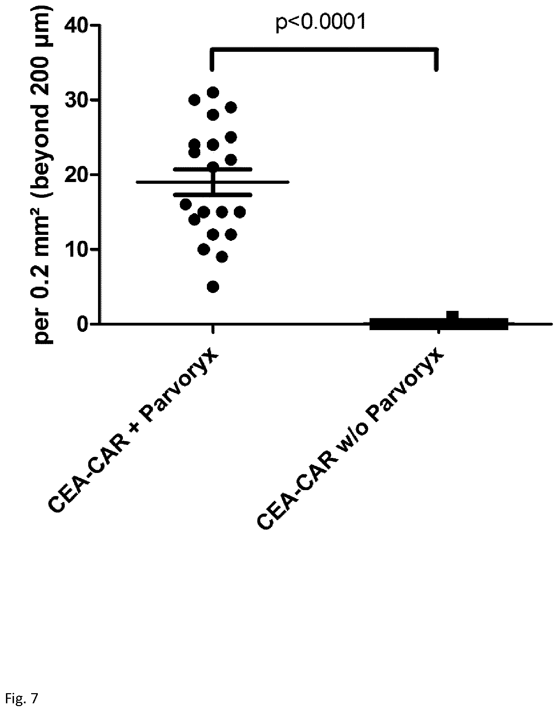

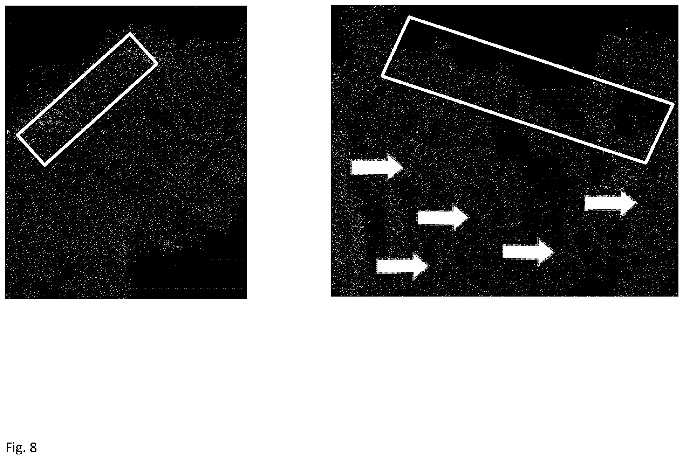

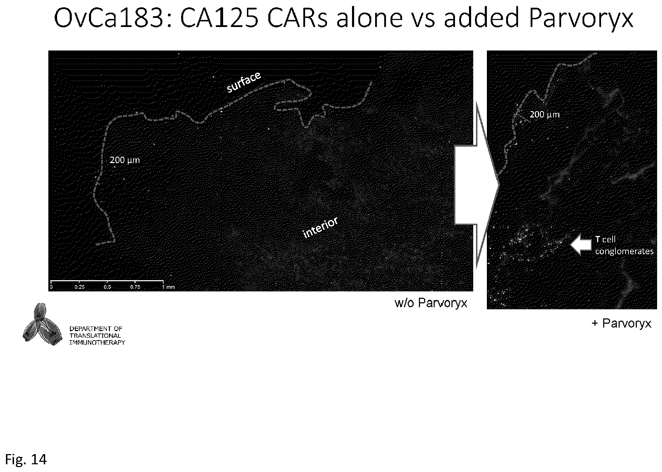

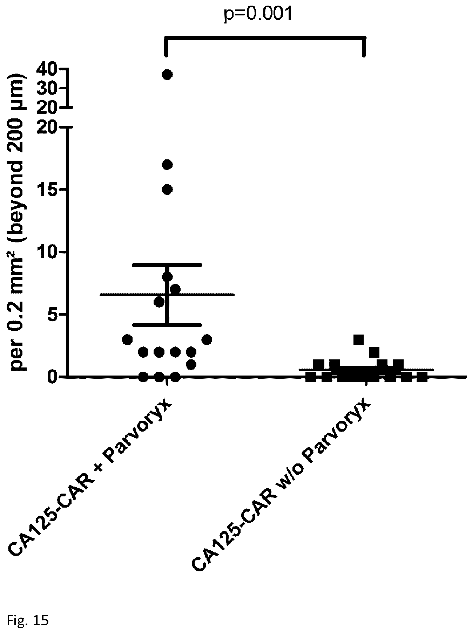

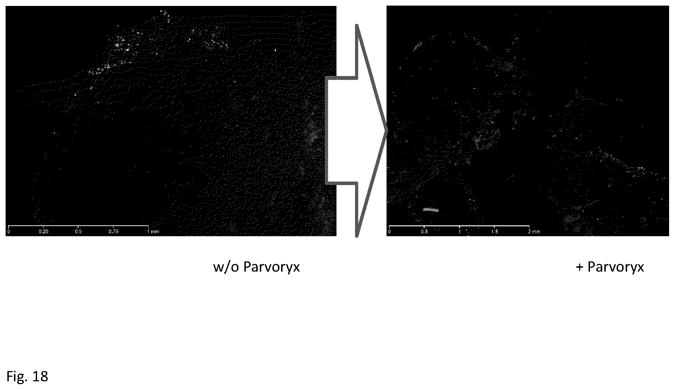

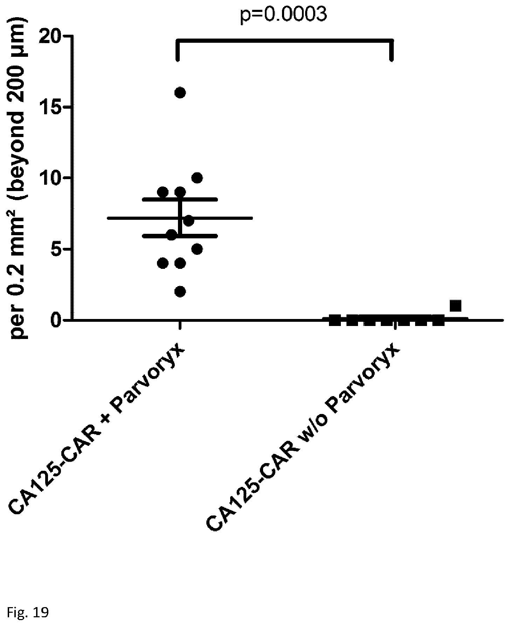

In addition, it is shown that there is a massive difference in deep infiltration of the tumor tissue occurs when parvovirus H-1 is present (Fig.6, 7, 8, 14, 15, 18, 19). Thus, in summary, the addition of parvovirus H-1 significantly improves infiltration of T cells. The infiltration of specific CAR-modified T cells is enhanced and supersedes simple T cell infiltration. Infiltrating specific CAR-T cells are activated, produce TH1 cytokines and chemokines for further infiltration which is most likely due to CARs engaging the tumor. This effect is not shown when mock-CARs are used that do not engage with the tumor.

The term "about" means approximately or nearly, and in the context of a numerical value or range set forth herein in one embodiment means ± 20%, ± 10%, ± 5%, or ± 3% of the numerical value or range recited or claimed.

The terms "a" and "an" and "the" and similar reference used in the context of describing the disclosure (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the disclosure and does not pose a limitation on the scope of the claims. No language in the specification should be construed as indicating any non-claimed element essential to the practice of the disclosure.

Unless expressly specified otherwise, the term "comprising" is used in the context of the present document to indicate that further members may optionally be present in addition to the members of the list introduced by "comprising". It is, however, contemplated as a specific embodiment of the present disclosure that the term "comprising" encompasses the possibility of no further members being present, i.e., for the purpose of this embodiment "comprising" is to be understood as having the meaning of "consisting of".

In addition, the figures, materials, methods, and examples are illustrative only and not intended to be limiting. Headings, sub-headings or numbered or lettered elements, e.g., (a), (b), (i) etc., are presented merely for ease of reading. The use of headings or numbered or lettered elements in this document does not require the steps or elements be performed in alphabetical order or that the steps or elements are necessarily discrete from one another. Other features, objects, and advantages of the invention will be apparent from the description and drawings, and from the claims.

### Brief Description of the Figures

In the Figures and following Examples "Parvoryx" means an administrable formulation containing wild-type parvovirus H-1.
Fig. 1: Human CRC-Liver Metastasis Ex-Vivo Cell Migration Analysis Model
Fig. 2: Principle of CAR-T therapy
Fig. 3: Generations of CAR cells
Fig. 4: Immunohistochemistry confirming CEA target structure positivity
Fig. 5: CMFDA labelled CAR transduced patient T cells were placed in the medium of the explant and explant was harvested 24h later. Subsequent fluorescent imaging shows the surface of the explant (colorectal cancer liver metastasis) being infiltrated up to a depth of 200 µm (white line).
Fig. 6: CMFDA labelled CEA-CAR transduced patient T cells after administration of ParvOryx (in medium) show enhanced massive infiltration beyond the 200 µm depth (= white line). Magnification shows CMFDA positive lymphocytes in the inner part of the colorectal cancer liver metastasis tissue.
Fig. 7: Density of specific CEA-CAR transduced patient T cells, comparing with or without previous application of ParvOryx virus (Mann Whitney non-parametric testing).
Fig. 8: Comparison of specific CEA-CAR versus mock CAR transduced T cell infiltration showing clear enhancing capabilities of infiltration with ParvOryx administration.
Fig. 9: Slight difference for specific CEA-CAR versus mock-CAR transduced T cell infiltration (Mann Whitney non-parametric test). ParvOryx administration enhances T cell infiltration independent of CAR-specificity.
Fig. 10: Cytokine level differences between specific CEA-CAR versus mock-CAR transduced T cell infiltrated tissues highlighting specific TH1-like activation patterns only for specific CAR transduced T cells.
Fig. 11: Cytokine level differences between specific CEA-CAR versus mock-CAR transduced T cell infiltrated tissues highlighting specific TH1-like activation patterns only for specific CAR transduced T cells.
Fig. 12: Cytokine level differences between specific CEA-CAR versus mock-CAR transduced T cell infiltrated tissues highlighting specific TH1-like activation patterns only for specific CAR transduced T cells.
Fig. 13: Percentage differences in tissue cytokines comparing specific CEA-CAR transduced T cell infiltrated and specific CEA-CAR transduced T cell infiltrated with concomitant ParvOryx administration. Asterisks mark specific infiltration-dependent cytokine modulations indicating specific T cell activation.
Fig. 14: CMFDA labelled CA125-CAR transduced patient T cells after administration of ParvOryx (medium) show enhanced massive infiltration beyond the 200 µm depth in ovarian cancer. Above part: w/o Parvovirus; Below part: + Parvovirus. Magnification shows CMFDA positive lymphocytes in the inner part of the ovarian cancer tissue (white arrow in the below part).
Fig. 15: Density of specific CA125-CAR transduced patient T cells, comparing with or without previous application of ParvOryx virus (Mann Whitney non-parametric testing).
Fig. 16: Cytokine level differences between specific CA125-CAR versus mock-CAR transduced T cell infiltrated tissues highlighting specific TH1-like activation patterns only for specific CAR transduced T cells.
Fig. 17: Cytokine level differences between specific CA125-CAR versus mock-CAR transduced T cell infiltrated tissues highlighting specific TH1-like activation patterns only for specific CAR transduced T cells, also for sustaining and differentiating signals like IL-7.
Fig. 18: CMFDA labelled CA125-CAR transduced patient T cells after administration of ParvOryx (medium) show enhanced massive infiltration beyond the 200 µm depth in ovarian cancer.
Fig. 19: Density of specific CA125-CAR transduced patient T cells, comparing with or without previous application of ParvOryx virus (Mann Whitney non-parametric testing).
Fig. 20: Cytokine level differences between specific CA125-CAR versus mock-CAR transduced T cell infiltrated tissues highlighting specific TH1-like activation patterns only for specific CAR transduced T cells.
Fig. 21: Cytokine level differences between specific CA125-CAR versus mock-CAR transduced T cell infiltrated tissues highlighting specific TH1-like activation patterns only for specific CAR transduced T cells.
Fig. 22: Cytokine level differences between specific CA125-CAR versus mock-CAR transduced T cell infiltrated tissues highlighting specific TH1-like activation patterns only for specific CAR transduced T cells, also for sustaining and differentiating signals like IL-5 and IL-7.
Fig. 23: Cytokine level changes induced by ParvOryx (as ratios over untreated control, mean values shown for three ovarian cancer explants at 24h of treatment) and also change in CD8 T cell density (last column). The above shown cytokines are identical to the findings in other cancer entities (colorectal cancer liver metastases and pancreatic cancer)

### EXAMPLES

### Patients:

### (1) Colorectal Cancer with liver metastases

- Patient with multiple lines of chemotherapy
- Resection of CRC liver metastases (CRCLM)
- CEA positive tumor cells
- Successful peripheral blood sampling and T cell extraction
- Successful CAR transfection (mock and CEA-specific CARs)

### (2) Ovarian Cancer (Advanced Stage) OvCa183

- Patient with first line of chemotherapy
- Resection of abdominal tumor manifestations (OVCA)
- CA125 positive tumor cells according to pathology report
- Successful peripheral blood sampling and T cell extraction
- Successful CAR transfection (mock and CA125-specific CARs)

### (3) Ovarian Cancer (Advanced Stage) OvCa184

- Patient after first line of chemotherapy
- Resection of abdominal tumor manifestations (OVCA)
- CA125 positive tumor cells confirmed by pathology
- Successful peripheral blood sampling and T cell extraction
- Successful CAR transfection (mock and CA125-specific CARs)

### Example 1: Ex-plant model

### Tissue Culture

Fresh resected tumor tissues (CRCLM or ovarian cancer) were directly transferred from the operating room to the laboratory in 0.9% sodium saline solution (Sigma-Aldrich) and on ice. By using forceps and a scalpel each tissue was placed in a petri dish and split into small pieces containing equal proportions of invasive margin. One piece of tissue was directly frozen and stored as control. For autologous T cell isolation, an additional piece of adjacent tissue (e.g. liver) was set apart. Tissue pieces were placed into 96-well plates and cultured under sterile conditions at 37 °C and 5% carbon dioxide in MEM containing 2.9% of 7.5% sodium bicarbonate solution and 1% of 200 mM L-Glutamine solution (Sigma-Aldrich). If necessary, pH of the culture media was adjusted to 7.4 using sodium hydroxide. After 24 to 72 h, tissues were harvested, placed into small plastic bowls (Sakura Finetek Germany GmbH) containing tissue embedding compound (VWR) and directly frozen in liquid nitrogen. Frozen tissues were stored at - 80 °C until further usage.

### T Cell Isolation and Labeling

For isolation of autologous T cells, an approximately 0.5 cm² piece of fresh resected human CRC-LM tissue (adjacent liver if available) was placed in a petri dish and minced with a scalpel. Sheared tissue was gently added with forceps on a cell strainer (40 µm mesh) and flushed into a 50 ml falcon tube using RPMI (Sigma-Aldrich). Flow-through was flushed a second time through a new cell strainer and isolated cells were stained with 5 µM CellTracker^{™} Green CMFDA (Thermo Fisher Scientific) for 1 h in cell culture flasks. Non-adherent T cells were isolated by negative bead-based isolation using the Dynabeads Untouched Human T Cells Kit (Thermo Fisher Scientific). Autologous CMFDA T cells were directly re-suspended in tissue culture medium for the ex vivo cell migration analysis.

Donor T cells were obtained from peripheral blood of healthy donors by density gradient centrifugation using Ficoll Paque Plus (Sigma-Aldrich) following 1.5 h separation of non-adherent cells in cell culture flasks and negative bead-based T cell isolation. To stimulate proliferation, isolated donor T cells were cultured for 48 h in Xvivo 15 medium (Biozym) with anti-human CD3 antibody (1:10.000, clone OKT3, BioLegend) and daily addition of 300 Units IL-2 (PeproTech). T cells were stained with 5 µM CMFDA for 1 h and cryopreserved in FBS (Biochrom) with 10% DMSO. Frozen CMFDA donor cells were stored at - 80 °C until usage for ex vivo cell migration analysis.

### Example 2: Generation of CAR-T cells

To obtain primary T cells, 50ml blood was taken from 3 healthy donors in EDTA collection tubes. The fresh blood was thoroughly pipetted onto a layer of 10ml Ficoll (Ficoll Paque^{™}, density: 1.077; GE Healthcare) and centrifuged at 750xg for 30min without brake. Lymphocytes were taken off and washed twice with PBS. Cell number was determined and isolation of CD3⁺ T cells was carried out using the human Pan T cell Isolation Kit II (Miltenyi Biotec) according to the manufacturer's instructions. The number of T cells was determined after sorting and 1x10⁶ /ml/cm² cells were seeded in activation medium (XVIVO20 (Lonza) containing 100ng/ml of an anti-CD3 antibody (Clone: OKT3; Janssen-Cilag) and 300U/ml Interleukin-2 (ProLeukin^{®}; Novartis)) for 24h at 37°C. After activation, T cells were washed three times in PBS and further incubated in cultivation medium (XVIVO20; 300U/ml IL-2). Fourty-eight hours after isolation of T cells, lentiviral CAR transduction was carried out using the spinoculation method. Therefore, cells were seeded on a Retronectin^{®}-coated 24well plate (16µg/ml Retronectin^{®}; 1x10⁶ CD3⁺ cells/ml/cm²) and lentiviral particles encoding the CAR gene expression cassette (4H11scFv-IgG-28BBz or SCA431scFV-IgG-28BBz) were added at an MOI of 10. The plate was centrifuged for 1.5h at 2000g and 32°C followed by incubation at 37°C for 24h. Thereafter, medium containing lentiviral particles was removed and replaced with cultivation medium. Seventy-two hours after initial cell sorting, the rate of CAR-expressing CD3⁺ cells was determined by flow cytometry. Detailed information to the different donors are given in the table below.

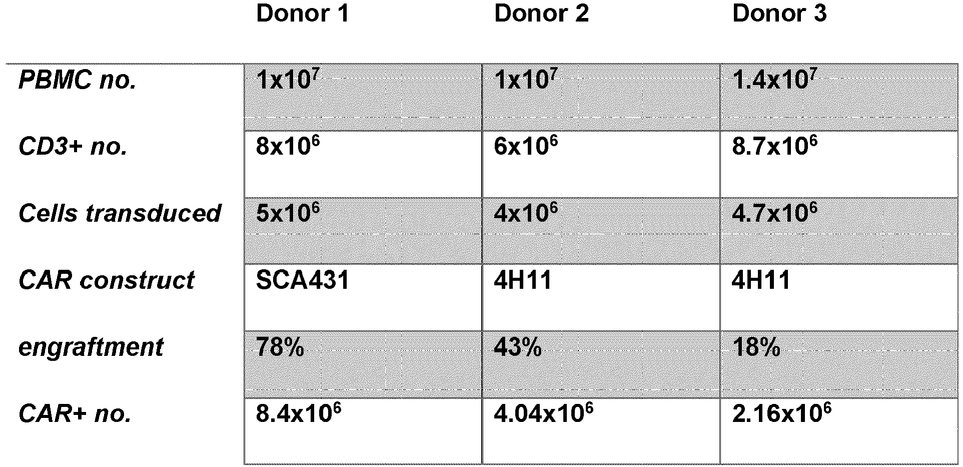

### Example 3: Treatment in ex-plant model

ParvOryx, i.e. a GMP-grade preparation of H-1PV, was used. H-1 PV was given in a concentration of 1 x 10⁷ pfu /ml into the tissue culture and after 60 minutes the above mentioned numbers of CAR cells were added and incubated for further 60 minutes.

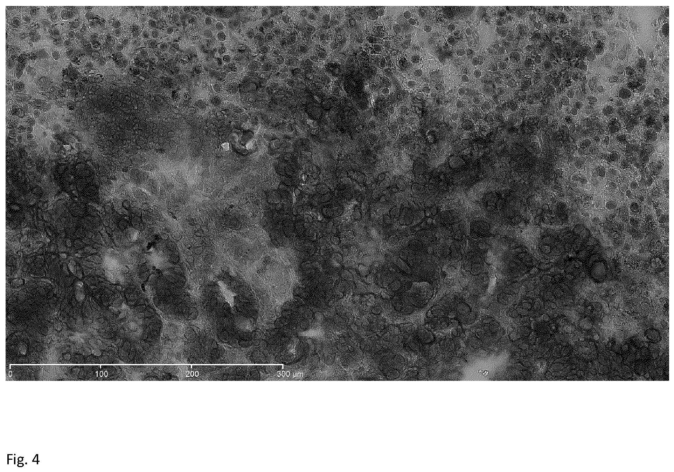

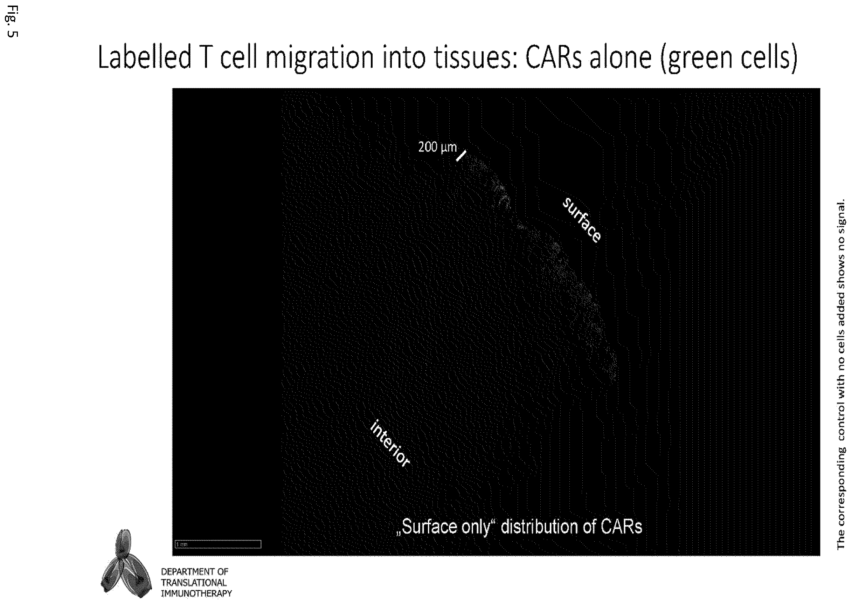

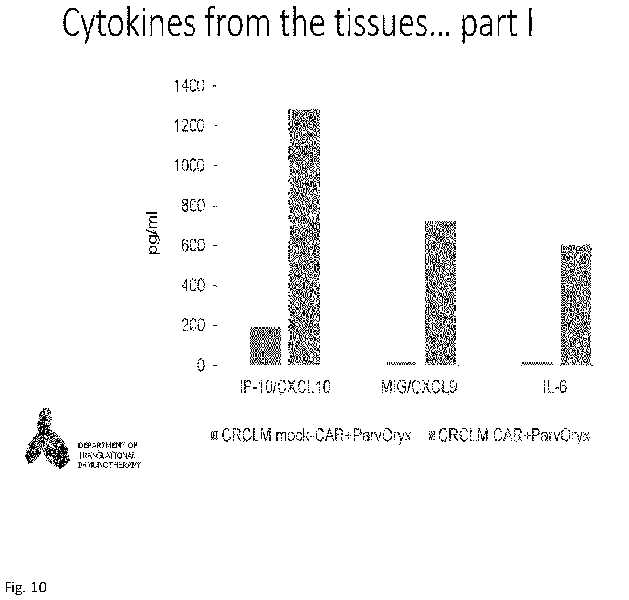

The explants treated with parvovirus H-1 and/or CAR-T cells or mock-CAR-T cells were then subjected to histological analysis (Fig. 4, 5, 6, 8) and multiplex protein analysis (Fig. 10, 11,12, 16, 17).

## Claims

1. Pharmaceutical combination containing (a) an oncolytic virus and (b) immune cells, particularly T cells, genetically modified to express a chimeric antigen receptor (CAR) specific for a cancer cell antigen.

2. The pharmaceutical combination of claim 1, wherein the oncolytic virus is parvovirus H-1 or a related rodent parvovirus selected from Lulll, Mouse minute virus (MMV), Mouse parvovirus (MPV), Rat minute virus (RMV), Rat parvovirus (RPV), or Rat virus (RV).

3. The pharmaceutical combination of claim 1, wherein the cancer cell antigen is selected from mesothelin, EGFRvIII, GD2, Tn antigen, PSMA, PSA, CD70, CD97, TAG72, CD44v6, CEA, CA125, EPCAM, KIT, IL-13Ra2, leguman, GD3, CD171, IL-I IRa, PSCA, MAD-CT- 1, MAD-CT-2, VEGFR2, LewisY, CD24, PDGFR-beta, SSEA-4, folate receptor alpha, ERBBs (e.g., ERBB2), Her2/neu, MUC1, EGFR, NCAM, Ephrin B2, CAIX, LMP2, sLe, HMWMAA, o-acetyl-GD2, folate receptor beta, TEM1/CD248, TEM7R, FAP, Legumain, HPV E6 or E7, p16^{INK4a}, ML-IAP, CLDN6, TSHR, GPRC5D, ALK, polysialic acid, Fos-related antigen, neutrophil elastase, TRP-2, CYP1B1, sperm protein 17, beta human chorionic gonadotropin, AFP, thyroglobulin, PLAC1, globoH, RAGE1, MN-CA IX, MSI frameshift mutants, human telomerase reverse transcriptase, intestinal carboxyl esterase, mut hsp 70-2, NA-17, NY-BR-1, UPK2, HAVCR1, ADRB3, PANX3, NY-ESO-1, GPR20, Ly6k, OR51E2, TARP, or GFRa4.

4. The pharmaceutical combination of claim 3, wherein the cancer cell antigen is CEA or CA125.

5. The pharmaceutical combination of any of claims 1-4, further comprising one or more additional therapeutic agents selected from chemotherapeutic agents, biotherapeutic agents, an immunogenic agents, immune stimulating cytokines and cells transfected with genes encoding immune stimulating cytokines.

6. The pharmaceutical combination as defined in any of claims 1-5 for use in a method of treating cancer.

7. The pharmaceutical combination for the use according to claim 6, wherein the oncolytic virus and the immune cells genetically modified to express a chimeric antigen receptor (CAR) specific for a cancer cell antigen are sequentially administered.

8. The pharmaceutical combination for the use according to claim 6 or 7, wherein the immune cells are T cells.

9. The pharmaceutical combination for the use according to any of claims 6 to 8, wherein the use is for treating solid tumours, haematological cancer and/or cancer initiating stem cells.

10. The pharmaceutical combination for use according to any of claims 6 to 9, wherein the cancer is colon cancer, bladder cancer, liver cancer, breast cancer, kidney cancer, head/neck squamous cell carcinoma, lung cancer, malignant melanoma, ovarian cancer, pancreatic cancer, prostate cancer, brain cancer, cervical cancer, renal cell cancer or stomach cancer.

11. The pharmaceutical composition for the use according to any one of claims 6 to 10, wherein the oncolytic virus and/or the CAR immune cells are administered by intratumoral or intravenous administration.

12. A kit which comprises a first container, a second container and a package insert, wherein the first container comprises at least one dose of a pharmaceutical composition containing an oncolytic virus, the second container comprises at least one dose of immune cells, preferably T cells, genetically modified to express a chimeric antigen receptor (CAR) specific for a cancer cell antigen, and the package insert comprises instructions for treating an individual having cancer.

13. The kit of claim 12, wherein the cancer is colon cancer, bladder cancer, liver cancer, breast cancer, kidney cancer, head/neck squamous cell carcinoma, lung cancer, malignant melanoma, ovarian cancer, pancreatic cancer, prostate cancer, brain cancer, cervical cancer, renal cell cancer or stomach cancer.

14. A method of inhibiting proliferation and/or activity of tumour antigen-positive cells in an individual, comprising the step of providing to the individual a therapeutically effective amount of a pharmaceutical combination of any of claims 1 to 5.

15. The method of claim 14, wherein the components (a) and (b) of the pharmaceutical combination are provided individually by intratumoral or intravenous administration.

16. An oncolytic virus for use in a method of increasing TH1 type cytokines, upregulating pro-migratory chemokines and upregulating tonic T cell stimulatory interleukines in the microenvironment of a tumor.

17. The oncolytic virus for the use of claim 16, wherein the oncolytic virus is parvovirus H-1 or a related rodent parvovirus selected from Lulll, Mouse minute virus (MMV), Mouse parvovirus (MPV), Rat minute virus (RMV), Rat parvovirus (RPV), or Rat virus (RV).
